# EUROPEAN PATENT APPLICATION

(11) **EP 3 460 687 A1**
(43) Date of publication of application: **27.03.2019**
(21) Application number: 17193302.1
(22) Date of filing: 26.09.2017
(51) Int. Cl.: G06F 19/00

(54) **A COMPUTER-IMPLEMENTED METHOD OF MONITORING A PERSON AND AN APPARATUS FOR MONITORING A PERSON**

(71) Applicant: Koninklijke Philips N.V., 5656 AE Eindhoven (NL)
(72) Inventor: BULUT, Murtaza, 5656 AE Eindhoven (NL); DE MASSARI, Daniele, 5656 AE Eindhoven (NL); DE VRIES, Jan Johannes Gerardus, 5656 AE Eindhoven (NL); VAN DE CRAEN, Dieter Maria Alfons, 5656 AE Eindhoven (NL)
(74) Representative: de Haan, Poul Erik

(57) **Abstract**

According to an aspect there is provided a computer-implemented method of monitoring a person, the method comprising (i) receiving medication schedule information regarding a person, the medication schedule information indicating the timing of medication doses for treating or suppressing one or more symptoms of a medical condition; (ii) setting a first monitoring interval for a first monitoring event for the person based on the received medication schedule, wherein the first monitoring interval starts before a first medication dose is scheduled to be taken in which a first monitoring event can be initiated; (iii) initiating a first monitoring event for the person in the first monitoring interval and monitoring the person for one or more characteristics associated with the one or more symptoms of the medical condition during the first monitoring event;(iv) setting a second monitoring interval for a second monitoring event for the person, wherein the second monitoring interval starts before a second medication dose is scheduled to be taken by the person in which a second monitoring event can be initiated, and wherein a start time, end time and/or duration of the second monitoring interval is determined based on the monitored one or more characteristics and the medication schedule.

## Description

### TECHNICAL FIELD OF THE INVENTION

The invention relates to a computer-implemented method of monitoring a person and an apparatus for monitoring a person, and in particular to a method and apparatus for scheduling a monitoring event used to determine the effectiveness of a medication.

### BACKGROUND to the Invention

At-home elderly care services is a fast growing market which enables elderly people to live independently in the comfort of their home, while still having access to medical support when needed. These services often include a video call system to enable remote interaction between the caregiver and elderly person. During a video call the caregiver can ask targeted questions with the aim of getting a complete overview of the elderly person's health status. For instance, topics that can be addressed include sleep, physical activity, nutrition intake, toilet behaviour, social interactions, cognition, and adherence to a medication schedule. The caregiver can also monitor how the person talks, how the person walks, their facial expressions, their body posture, etc.

Parkinson's disease (PD) is a brain disorder that progressively affects a person's ability to control body movements, caused by a disorder of certain nerve cells in a part of the brain that produces dopamine, a chemical messenger the brain uses to help direct and control body movement. As these nerve cells break down, dopamine levels drop and brain signals that direct movement become abnormal. Symptoms of Parkinson's disease appear when these dopamine-producing nerve cells become damaged or die. Various domains of autonomic function, including sudomotor, cardiovagal, and adrenergic functions are impaired in people with PD.

The main symptoms of Parkinson's disease are tremors or trembling of a limb, especially when the body is at rest, slow movement, an inability to move, rigid limbs, a shuffling gait, and stooped posture. It also produces a wide range of other problems for people, amongst others: problems with swallowing and chewing, speech impairments, urinary problems or constipation, excessive sweating and other skin problems, depression and other emotional changes, and difficulties with sleep.

The main medications (or drugs) for Parkinson's disease are levodopa (or L-Dopa) and Dopamine Agonists. Levodopa is the gold standard, and it works by replacing the dopamine: in the brain neurons convert levodopa into dopamine. One of the problems with levodopa is the drug regime management, as prediction of the 'on' and 'off' periods of Parkinson's disease becomes more difficult and unpredictable with the disease progression. Dopamine agonists work by stimulating the parts of the brain that are influenced by dopamine, tricking the brain that it received dopamine. They are not as effective as levodopa, and are prescribed in the very early stages or as complement to levodopa.

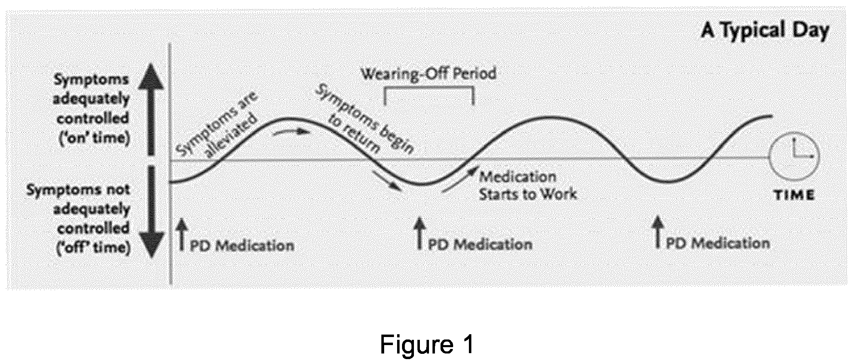

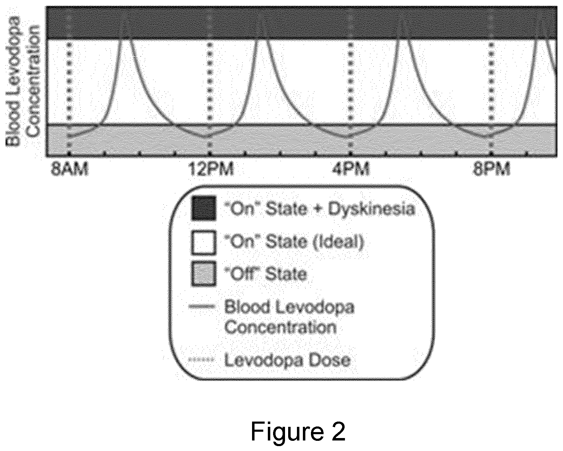

The plasma concentration of levodopa (and therefore the drug intake time, and amount) is linked to the on/off state as shown in Figures 1 and 2. Figure 1 is a graph showing the presence or absence of symptoms over time, and thus shows the relationship between symptom control, medication dose/frequency and a 'wearing off' period in which the symptoms start to return. Figure 2 shows levodopa concentration in the blood over time. Thus, it can be seen from both graphs that there can be periods between medication doses where the drug levels are sufficient and the person is in an 'on' state where the PD symptoms are controlled (i.e. not present or minimised) and periods where drug levels are not sufficient and the person is in an 'off' state (where PD symptoms such as trembling, and speech-related problems return, or become present). There can also be periods where the drug concentration is too high, and this can result in dyskinesia, which manifests as motion, speech, and sweating-related problems. Avoiding or minimising the 'off' states and minimising the 'over dosing' states by finding an appropriate medication regimen (i.e. frequency and quantity) is one of the biggest problems and challenges in the current PD treatment.

Finding the right dosage is very difficult because the right dosage is different for every person, and the right dosage significantly changes with the progression of the disease. In particular, when the disease progresses the time interval between 'on' and 'off' periods decreases (with the duration of on periods decreasing and the duration of off periods increasing, in other words the off periods return faster), indicating a need for the dosage and/or frequency of the medication to be increased. Current approaches for finding the right dosage of PD medications are currently based on trial and error.

There are similar problems with other diseases and conditions that have symptoms that can be controlled using an appropriate medication regimen, and where symptoms can return if the medication regimen is not correct for the person, non-limiting list of examples is heart failure, usage of blood thinners, depression, osteoarthritis and epilepsy.

Establishing the right medication regimen for a person can be even more difficult when a person is monitored using at-home monitoring services, since there might not be frequent contact with the person, and it can be difficult to determine the extent to which a medication regimen is working or not during the video call, particularly if the video call takes place during an 'on' state for PD when the symptoms are well controlled. It is undesirable to continuously monitor a person, particularly where the monitoring involves interactions with a remote care provider or involves the person being observed using a camera, since this is intrusive to the person.

Therefore there is a need for improvements in the monitoring of a person, and in particular improvements in the scheduling of monitoring events in which the person is monitored for symptoms of a disease, to enable better decisions to be taken on a medication regimen for the person, and also (or alternatively) to collect information on the effectiveness of a medication regimen.

### SUMMARY OF THE INVENTION

According to a first aspect, there is provided a computer-implemented method of monitoring a person (i.e. a patient, a subject, a user, an individual), the method comprising (i) receiving medication schedule information regarding the person, the medication schedule information indicating the timing of medication doses for treating or suppressing one or more symptoms of a medical condition; (ii) setting a first monitoring interval for a first monitoring event for the person based on the received medication schedule, wherein the first monitoring interval starts before a first medication dose is scheduled to be taken in which a first monitoring event can be initiated; (iii) initiating a first monitoring event for the person in the first monitoring interval and monitoring the person for one or more characteristics associated with the one or more symptoms of the medical condition during the first monitoring event; (iv) setting a second monitoring interval for a second monitoring event for the person, wherein the second monitoring interval starts before a second medication dose is scheduled to be taken by the person in which a second monitoring event can be initiated, and wherein a start time, end time and/or duration of the second monitoring interval is determined based on the monitored one or more characteristics and the medication schedule. Thus, the first aspect provides that the scheduling of subsequent monitoring events can take into account the presence or absence of symptoms of the medical condition relative to a scheduled medication dose in order to provide better information on the effectiveness of a medication regimen.

In some embodiments, if the monitored one or more characteristics indicate that the person did not have the one or more symptoms of the medical condition during the first monitoring event, the start time of the second monitoring interval is determined to be (i) the same amount of time before the time at which the second medication dose is scheduled to be taken than the start time of the first monitoring interval was to the time at which the first medication dose was scheduled to be taken, or (ii) closer to the time at which the second medication dose is scheduled to be taken than the start time of the first monitoring interval was to the time at which the first medication dose was scheduled to be taken. Thus, this embodiment provides that in the absence of symptoms during a monitoring event, a subsequent monitoring event is more likely to occur closer to the time of the next scheduled medication dose, increasing the chance of detecting a time point at which symptoms reoccur.

In some embodiments, if the monitored one or more characteristics indicate that the person did not have the one or more symptoms of the medical condition during the first monitoring event, the start time of the second monitoring interval is determined to be (i) at a time before the second medication dose is scheduled to be taken so that the time between the start time of the second monitoring interval and the time that the second medication dose is scheduled to be taken is equal to or less than the time between the time that the first medication dose was scheduled to be taken and the time that the first monitoring event ended, provided that the time that the first monitoring event ended was before the time that the first medication dose was scheduled to be taken; and (ii) at a time before the second medication dose is scheduled to be taken so that the time between the start time of the second monitoring interval and the time that the second medication dose is scheduled to be taken is equal to or less than the time between the time that the first medication dose was scheduled to be taken and the time that the first monitoring event started, provided that the time that the first monitoring event ended was after the time that the first medication dose was scheduled to be taken. Thus, this embodiment provides that in the absence of symptoms during a monitoring event, a subsequent monitoring event will occur closer to the time of the next scheduled medication dose, increasing the chance of detecting a time point at which symptoms reoccur.

In some embodiments, the end time of the second monitoring interval is determined to be (i) a determined duration of time after the determined start time of the second monitoring interval, or (ii) the time that the second medication dose is scheduled to be taken, whichever is earlier. This embodiment ensures that, even with the monitoring interval having a preferred or predetermined duration, a monitoring event cannot start after the time at which a medication dose is scheduled to be taken.

In some embodiments, if the monitored one or more characteristics indicate that the person did have the one or more symptoms of the medical condition during the first monitoring event, the end time of the second monitoring interval is determined to be (i) the same amount of time before the time at which the second medication dose is scheduled to be taken than the end time of the first monitoring interval was to the time at which the first medication dose was scheduled to be taken, or (ii) further from the time at which the second medication dose is scheduled to be taken than the end time of the first monitoring interval was to the time at which the first medication dose was scheduled to be taken. Thus, this embodiment provides that in the presence of symptoms during a monitoring event, subsequent monitoring events are more likely to occur further away from the time of the next scheduled medication dose, increasing the chance of detecting a time point at which symptoms do not reoccur.

In some embodiments, if the monitored one or more characteristics indicate that the person did have the one or more symptoms of the medical condition during the first monitoring event, the end time of the second monitoring interval is determined to be at a time before the second medication dose is scheduled to be taken so that the time between the end time of the second monitoring interval and the time that the second medication dose is scheduled to be taken is equal to or more than the time between the time that the first medication dose was scheduled to be taken and the time that the first monitoring event was started. Thus, this embodiment provides that in the presence of symptoms during a monitoring event, subsequent monitoring events will occur further away from the time of the next scheduled medication dose, increasing the chance of detecting a time point at which symptoms do not reoccur.

In some embodiments, the start time of the second monitoring interval is a determined duration of time before the determined end time of the second monitoring interval.

In some embodiments, the method further comprises the step of determining a severity of the one or more symptoms based on the monitored one or more characteristics; and the duration of the second monitoring interval is determined based on the determined severity of the one or more symptoms. This embodiment recognises that if the symptoms are severe then the monitoring event should have occurred much earlier in time to identify a time point at which the symptoms started to reoccur, and so subsequent monitoring intervals can be set longer to increase the chance of those earlier monitoring events occurring.

In these embodiments, the duration of the second monitoring interval can be set longer than a duration of the first monitoring interval if the severity of the one or more symptoms is above a first threshold.

In these embodiments, the duration of the second monitoring interval is set the same as a duration of the first monitoring interval if the severity of the one or more symptoms is below a second threshold. In some embodiments, the first threshold is the same as the second threshold.

In some embodiments, the step of monitoring the person for one or more characteristics comprises processing an audio and/or video signal, and/or a sensor measurement signal obtained during the first monitoring event to determine the one or more characteristics. These embodiments provide that the symptoms can be observed automatically and consistently across different monitoring events.

In some embodiments, the one or more characteristics comprise characteristics of the speech of the person, characteristics of the movement of the person and/or physiological characteristics of the person.

In some embodiments, the step of monitoring the person for one or more characteristics associated with the one or more symptoms of the medical condition comprises receiving an input from a party involved in the monitoring event indicating the presence, absence and/or severity of the one or more symptoms during the first monitoring event.

In alternative embodiments, the step of monitoring the person for one or more characteristics associated with the one or more symptoms of the medical condition comprises activating or adapting the operation of one or more sensors to measure the one or more characteristics. These embodiments provide that sensors on the person or in the person's environment are only activated or used during a monitoring event, thereby minimising power consumption (particularly for sensors are battery powered), and/or reducing privacy concerns for the person.

In some embodiments, the method further comprises the step of receiving information indicating a time at which the person took the first medication dose. This embodiment enables the medication dose time to be compared to the information indicating whether or not symptoms occurred during the monitoring event to determine whether the dose was scheduled at an appropriate time.

In some embodiments, the method further comprises the step of estimating a time at which the person took the first medication dose from measurements of the one or more characteristics associated with the one or more symptoms of the medical condition.

In some embodiments, the start time of the second monitoring interval, the end time of the second monitoring interval and/or duration of the second monitoring interval is determined based on the monitored one or more characteristics, the medication schedule and the time at which the person took the first medication dose.

In some embodiments, the method further comprises the step of calculating or adjusting the timing and/or dosage of medication doses in the medication schedule based on the monitored one or more characteristics and the time at which the person took the first medication dose. Thus, this embodiment provides that information obtained during a monitoring event can be used to inform decisions about whether to change a medication dosage and/or medication schedule.

In some embodiments, the step of setting the second monitoring interval comprises increasing the duration of the second monitoring interval relative to a duration of the first monitoring interval if the timing and/or dosage of the medication doses in the medication schedule is adjusted. This embodiment is useful since the effect of the change in medication timing and dosage is not certain, and so provides a wider range of times at which a subsequent monitoring event can occur.

In some embodiments, the first monitoring interval has an end time that corresponds to the time at which the first medication dose is scheduled to be taken.

In some embodiments, the duration of the first monitoring interval is set based on an estimate of when the one or more symptoms may occur following an earlier medication dose.

In some embodiments, the method further comprises the step of repeating steps (iii) and (iv) for the second monitoring event and second medication dose to set a third monitoring interval for a third monitoring event to the person, wherein the third monitoring interval starts before a third medication dose is to be taken by the person and is an interval in which a third monitoring event can be initiated.

In some embodiments, the method further comprises the step of repeating steps (iii) and (iv) for the second monitoring event and subsequent monitoring events to collect information on the influence of the medication doses on the one or more symptoms of the medical condition.

In some embodiments, the medical condition is Parkinson's Disease, heart failure, usage of blood thinners, depression, epilepsy or osteoarthritis.

In some embodiments, the monitoring event is a telephone call, a video call, an interaction by the person with an electronic device, or the monitoring of the person using one or more electronic devices.

According to a second aspect, there is provided a computer program product comprising a computer readable medium having computer readable code embodied therein, the computer readable code being configured such that, on execution by a suitable computer or processor, the computer or processor is caused to perform any of the methods described above or herein.

According to a third aspect, there is provided an apparatus for monitoring a person, the apparatus comprising a processing unit (or a control unit) configured to receive medication schedule information regarding a person, the medication schedule information indicating the timing of medication doses for treating or suppressing one or more symptoms of a medical condition; set a first monitoring interval for a first monitoring event for the person based on the received medication schedule, wherein the first monitoring interval starts a time period before a first medication dose is scheduled to be taken in which a first monitoring event can be initiated; initiate a first monitoring event for the person in the first monitoring interval and monitoring the person for one or more characteristics associated with the one or more symptoms of the medical condition during the first monitoring event; and set a second monitoring interval for a second monitoring event for the person, wherein the second monitoring interval starts a time period before a second medication dose is scheduled to be taken by the person in which a second monitoring event can be initiated, and wherein a start time, end time and/or duration of the second monitoring interval is determined based on the monitored one or more characteristics and the medication schedule. Thus, the third aspect provides that the scheduling of subsequent monitoring events can take into account the presence or absence of symptoms of the medical condition relative to a scheduled medication dose in order to provide better information on the effectiveness of a medication regimen.

In some embodiments, the processing unit can be configured such that, if the monitored one or more characteristics indicate that the person did not have the one or more symptoms of the medical condition during the first monitoring event, the start time of the second monitoring interval is determined to be (i) the same amount of time before the time at which the second medication dose is scheduled to be taken than the start time of the first monitoring interval was to the time at which the first medication dose was scheduled to be taken, or (ii) closer to the time at which the second medication dose is scheduled to be taken than the start time of the first monitoring interval was to the time at which the first medication dose was scheduled to be taken. Thus, this embodiment provides that in the absence of symptoms during a monitoring event, a subsequent monitoring event is more likely to occur closer to the time of the next scheduled medication dose, increasing the chance of detecting a time point at which symptoms reoccur.

In some embodiments, the processing unit can be configured such that, if the monitored one or more characteristics indicate that the person did not have the one or more symptoms of the medical condition during the first monitoring event, the start time of the second monitoring interval is determined to be (i) at a time before the second medication dose is scheduled to be taken so that the time between the start time of the second monitoring interval and the time that the second medication dose is scheduled to be taken is equal to or less than the time between the time that the first medication dose was scheduled to be taken and the time that the first monitoring event ended, provided that the time that the first monitoring event ended was before the time that the first medication dose was scheduled to be taken; and (ii) at a time before the second medication dose is scheduled to be taken so that the time between the start time of the second monitoring interval and the time that the second medication dose is scheduled to be taken is equal to or less than the time between the time that the first medication dose was scheduled to be taken and the time that the first monitoring event started, provided that the time that the first monitoring event ended was after the time that the first medication dose was scheduled to be taken. Thus, this embodiment provides that in the absence of symptoms during a monitoring event, a subsequent monitoring event will occur closer to the time of the next scheduled medication dose, increasing the chance of detecting a time point at which symptoms reoccur.

In some embodiments, the processing unit can be configured to determine the end time of the second monitoring interval as (i) a determined duration of time after the determined start time of the second monitoring interval, or (ii) the time that the second medication dose is scheduled to be taken, whichever is earlier. This embodiment ensures that, even with the monitoring interval having a preferred or predetermined duration, a monitoring event cannot start after the time at which a medication dose is scheduled to be taken.

In some embodiments, the processing unit can be configured such that, if the monitored one or more characteristics indicate that the person did have the one or more symptoms of the medical condition during the first monitoring event, the end time of the second monitoring interval is determined to be (i) the same amount of time before the time at which the second medication dose is scheduled to be taken than the end time of the first monitoring interval was to the time at which the first medication dose was scheduled to be taken, or (ii) further from the time at which the second medication dose is scheduled to be taken than the end time of the first monitoring interval was to the time at which the first medication dose was scheduled to be taken. Thus, this embodiment provides that in the presence of symptoms during a monitoring event, subsequent monitoring events are more likely to occur further away from the time of the next scheduled medication dose, increasing the chance of detecting a time point at which symptoms do not reoccur.

In some embodiments, the processing unit can be configured such that, if the monitored one or more characteristics indicate that the person did have the one or more symptoms of the medical condition during the first monitoring event, the end time of the second monitoring interval is determined to be at a time before the second medication dose is scheduled to be taken so that the time between the end time of the second monitoring interval and the time that the second medication dose is scheduled to be taken is equal to or more than the time between the time that the first medication dose was scheduled to be taken and the time that the first monitoring event was started. Thus, this embodiment provides that in the presence of symptoms during a monitoring event, subsequent monitoring events will occur closer further away from the time of the next scheduled medication dose, increasing the chance of detecting a time point at which symptoms do not reoccur.

In some embodiments, the processing unit can be configured to determine the start time of the second monitoring interval a determined duration of time before the determined end time of the second monitoring interval.

In some embodiments, the processing unit can be further configured to determine a severity of the one or more symptoms based on the monitored one or more characteristics; and to determine the duration of the second monitoring interval based on the determined severity of the one or more symptoms. This embodiment recognises that if the symptoms are severe then the monitoring event should have occurred much earlier in time to identify a time point at which the symptoms started to reoccur, and so subsequent monitoring intervals can be set longer to increase the chance of those earlier monitoring events occurring.

In these embodiments, the processing unit can be configured to set the duration of the second monitoring interval longer than a duration of the first monitoring interval if the severity of the one or more symptoms is above a first threshold.

In these embodiments, the processing unit can be configured to set the duration of the second monitoring interval the same as a duration of the first monitoring interval if the severity of the one or more symptoms is below a second threshold. In some embodiments, the first threshold is the same as the second threshold.

In some embodiments, the processing unit can be configured to monitor the person for one or more characteristics by processing an audio and/or video signal, and/or a sensor measurement signal obtained during the first monitoring event to determine the one or more characteristics. These embodiments provide that the symptoms can be observed automatically and consistently across different monitoring events.

In some embodiments, the one or more characteristics comprise characteristics of the speech of the person, characteristics of the movement of the person and/or physiological characteristics of the person.

In some embodiments, the processing unit can be configured to monitor the person for one or more characteristics associated with the one or more symptoms of the medical condition by receiving an input from a party involved in the monitoring event indicating the presence, absence and/or severity of the one or more symptoms during the first monitoring event.

In alternative embodiments, the processing unit can be configured to monitor the person for one or more characteristics associated with the one or more symptoms of the medical condition by activating or adapting the operation of one or more sensors to measure the one or more characteristics. These embodiments provide that sensors on the person or in the person's environment are only activated or used during a monitoring event, thereby minimising power consumption (particularly for sensors are battery powered), and/or reducing privacy concerns for the person.

In some embodiments, the processing unit can be further configured to receive information indicating a time at which the person took the first medication dose. This embodiment enables the medication dose time to be compared to the information indicating whether or not symptoms occurred during the monitoring event to determine whether the dose was scheduled at an appropriate time.

In some embodiments, the processing unit can be further configured to estimate a time at which the person took the first medication dose from measurements of the one or more characteristics associated with the one or more symptoms of the medical condition.

In some embodiments, the processing unit can be further configured to determine the start time of the second monitoring interval, the end time of the second monitoring interval and/or duration of the second monitoring interval based on the monitored one or more characteristics, the medication schedule and the time at which the person took the first medication dose.

In some embodiments, the processing unit can be further configured to calculate or adjust the timing and/or dosage of medication doses in the medication schedule based on the monitored one or more characteristics and the time at which the person took the first medication dose. Thus, this embodiment provides that information obtained during a monitoring event can be used to inform decisions about whether to change a medication dosage and/or medication schedule.

In some embodiments, the processing unit can be configured to set the second monitoring interval by increasing the duration of the second monitoring interval relative to a duration of the first monitoring interval if the timing and/or dosage of the medication doses in the medication schedule is adjusted. This embodiment is useful since the effect of the change in medication timing and dosage is not certain, and so provides a wider range of times at which a subsequent monitoring event can occur.

In some embodiments, the processing unit can be configured to set an end time of the first monitoring interval that corresponds to the time at which the first medication dose is scheduled to be taken.

In some embodiments, the processing unit can be configured to set the duration of the first monitoring interval based on an estimate of when the one or more symptoms may occur following an earlier medication dose.

In some embodiments, the processing unit can be further configured to repeat (iii) and (iv) for the second monitoring event and second medication dose to set a third monitoring interval for a third monitoring event to the person, wherein the third monitoring interval starts before a third medication dose is to be taken by the person and is an interval in which a third monitoring event can be initiated.

In some embodiments, the processing unit can be further configured to repeat (iii) and (iv) for the second monitoring event and subsequent monitoring events to collect information on the influence of the medication doses on the one or more symptoms of the medical condition.

In some embodiments, the medical condition is Parkinson's Disease, heart failure, usage of blood thinners, depression, epilepsy or osteoarthritis.

In some embodiments, the monitoring event is a telephone call, a video call, an interaction by the person with an electronic device, or the monitoring of the person using one or more electronic devices.

These and other aspects of the invention will be apparent from and elucidated with reference to the embodiment(s) described hereinafter.

### BRIEF DESCRIPTION OF THE DRAWINGS

Exemplary embodiments of the invention will now be described, by way of example only, with reference to the following drawings, in which:
Figure 1 is a graph illustrating the presence or absence of symptoms over time;
Figure 2 shows levodopa concentration in the blood over time;
Figure 3 is a block diagram of an exemplary apparatus in which the invention can be implemented;
Figure 4 is a flow chart illustrating a method of monitoring a person according to an aspect;
Figures 5(a) and (b) illustrate two examples of setting first and second monitoring intervals according to embodiments of the invention; and
Figure 6 is a graph illustrating exemplary call intervals and calls according to an embodiment.

### DETAILED DESCRIPTION OF THE PREFERRED EMBODIMENTS

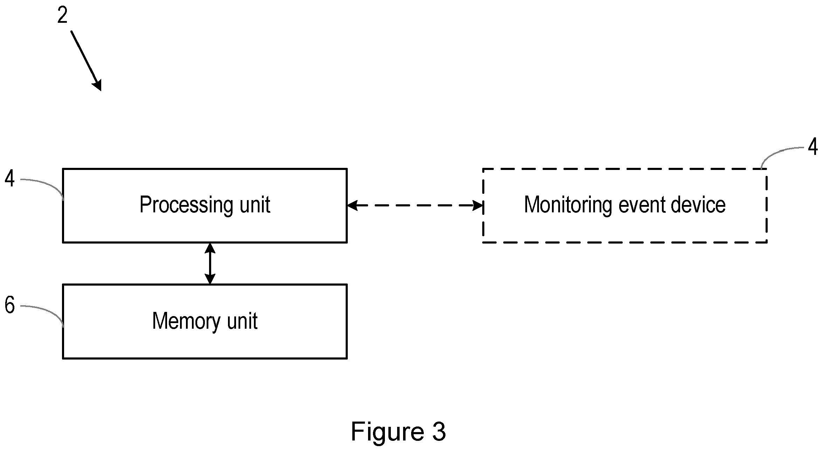

An exemplary apparatus 2 that can be used to implement the invention is shown in Figure 3. The apparatus 2 comprises a processing unit 4 and a memory unit 6. The processing unit 4 controls the operation of the apparatus 2 and can perform the steps of the methods described herein. The processing unit 4 can also be referred to as a control unit.

The processing unit 4 can be implemented in numerous ways, with software and/or hardware, to perform the various functions described herein. The processing unit 4 may comprise one or more microprocessors or digital signal processor (DSPs) that may be programmed using software or computer program code to perform the required functions and/or to control components of the processing unit 4 to effect the required functions. The processing unit 4 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

The memory unit 6 can comprise any type of memory, such as cache or system memory including volatile and non-volatile computer memory such as random access memory (RAM) static RAM (SRAM), dynamic RAM (DRAM), read-only memory (ROM), programmable ROM (PROM), erasable PROM (EPROM), and electrically erasable PROM (EEPROM).

The memory unit 6 can store program code that can be executed by the processing unit 4 to cause the processing unit 4 to perform the methods described herein. The memory unit 6 can also store information or signals for use during the methods described herein, for example, but not limited to, medication schedule information for one or more people, with the medication schedule information indicating the timing of medication doses for treating or suppressing one or more symptoms of a medical condition of the person.

The apparatus 2 can be any type of computing device, such as a computer, a server, a client node or device, a set-top box, a laptop, a tablet, a smart phone, etc. In some embodiments the apparatus 2 is a device that is remote from the person to be monitored (e.g. located in a healthcare facility whereas the person is at home). In this case, the apparatus 2 can be used to provide a monitoring service for monitoring a large number of people, in which case the apparatus 2 can be configured or arranged to perform the methods described herein for each of those people. In other embodiments, the apparatus 2 is a device that can be used, for example, in the home environment of the person, and is just used to monitor that person (and any other people in the same home).

It will be appreciated that the apparatus 2 can comprise additional components to those shown in Figure 3. For example, the apparatus 2 can comprise a power supply, such as a battery, or a connector or plug for enabling a connection to a mains power supply for powering the processing unit 4 and the memory unit 6. As another example, the apparatus 2 can comprise communications circuitry that can be used for one-way or two-way communications between the apparatus 2 and another electronic device, for example to receive information required for the execution of the methods described herein from another device (such as a medication schedule for the person), and/or for sending information resulting from the methods described herein to another device (such as information obtained during a monitoring event). As yet another example, the apparatus 2 can comprise a user interface for enabling a user of the apparatus 2 (e.g. the person or a care provider) to input commands and/or information into the apparatus 2, and/or to enable the apparatus 2 to provide information to a user. The user interface can comprise one or more user interface components, such as a display screen, one or more speakers, one or more lights, a keyboard, a keypad, a touch pad, a touch screen, a mouse, and a stylus, etc.

In addition to the processing unit 4 and the memory unit 6, Figure 3 shows a monitoring event device 8. The monitoring event device 8 can be part of the apparatus 2 or it can be separate from the apparatus 2. As such, the monitoring event device 8 can be remote from the apparatus 2, and/or local to the person. The monitoring event device 8 is used to perform a monitoring event on or with the person. In some embodiments, the monitoring event device 8 is a camera or similar device for enabling a video call, a telephone (including a mobile telephone or telephone headset) for enabling a voice call, one or more sensors for measuring one or more characteristics of the person that can be carried or worn by the person and/or be located in the environment around the person for monitoring the person. Where the one or more sensors are located in the environment, the sensor(s) may monitor one or more environmental parameters and/or parameters of the person. The one or more sensors may be controlled so that they only monitor the person during the monitoring event (i.e. the one or more sensors do not measure or monitor the person when a monitoring event is not occurring). Alternatively, if the one or more sensors are also used to monitor the person at times other than during a monitoring event (e.g. for purposes other than identifying symptoms of the medical condition), then the monitoring event can comprise increasing the accuracy of the monitoring, for example by increasing a sampling rate, by increasing the processing resources applied to the measurement signal to determine the characteristics, etc. In the case of a video call or telephone call, the monitoring event device 8 may comprise suitable software and/or hardware for processing the video stream or audio stream to determine measurements of one or more characteristics of the person during the call. In other embodiments, the processing unit 4 may perform this processing.

As noted above, establishing the right medication regimen for a person can be difficult when a person is monitored using at-home monitoring services, since there might not be frequent contact with the person, and it can be difficult to determine the extent to which a medication regimen is working or not during a video or voice call, particularly if the call takes place during a time when the symptoms of the person are being well controlled. It is undesirable to continuously monitor a person, particularly where the monitoring involves interactions with a remote care provider or involves the person being observed using a camera, since this is intrusive to the person. Therefore, the invention provides that the timing of a monitoring event (e.g. a voice or video call) is set based on the medication schedule of the person and an expected 'wearing off' time (or ineffective time) of the medication so that the effectiveness of the medication in the person can be monitored, with the timing of subsequent monitoring events being updated based on the person's condition or status during a monitoring event. With the invention, the monitoring of a person for symptoms of a disease is improved and thus enables better decisions to be taken on a medication regimen for the person (e.g. whether the dosage needs to be increased or decreased, or the frequency of doses needs to be adjusted), and also (or alternatively) to collect information on the effectiveness of a medication schedule, whether for a single person, or a population of people.

In an exemplary embodiment, which is particularly applicable to people with Parkinson's disease, the invention provides that regular (video) calls are timed (scheduled) for around the expected wearing off time of a drug or medication (e.g. levodopa) so that during the call the appropriateness of the dosage can be investigated. During the call the speech characteristics (e.g. loudness, intelligibility) and motion (e.g. trembling) of the person, as indicators of symptoms of the disease, can be easily measured. The only required input is the drug regimen of the person indicating when the person is scheduled to take doses of medication. A call typically takes place before a dose is scheduled to be taken, and based on whether the person showed symptoms of the disease during the call, a subsequent call can be scheduled closer to the next scheduled dose or further away (i.e. earlier) from the next scheduled dose. The medication schedule can be obtained from the person, a care provider or from a pill dispenser for the person, for example, and if it is known that the person takes the drugs every 4 hours at 8am, 12pm, 4pm, etc., then the invention can provide that a call is made with the person around one of these times, e.g. at 7:45am. In that way it is possible during the call to measure if the drug is wearing off faster than expected (which can happen with the progression of certain diseases, such as Parkinson's disease) and if so, to determine that the medication amount or medication itself may need to be changed.

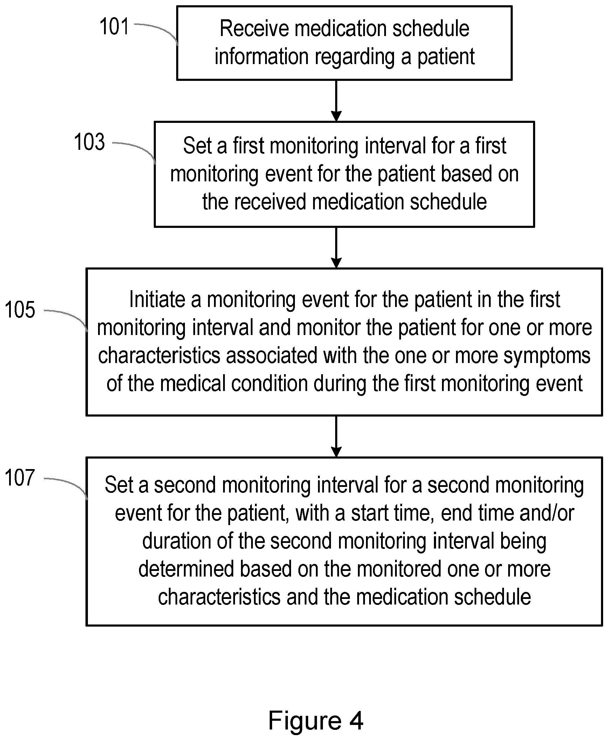

The flow chart in Figure 4 illustrates a method of monitoring a person according to an aspect. In some embodiments, the method is performed by the processing unit 4, for example as a result of executing suitable program code that is stored in the memory unit 6.

In a first step, step 101, medication schedule information for a person is received. The medication schedule information indicates the timing of medication doses for treating or suppressing one or more symptoms of a medical condition. The medication schedule may also indicate the amount of medication in each dose (e.g. a number of tablets, or weight of an active ingredient).

The medical condition can be any type of medical condition, and for example including Parkinson's disease, depression, heart failure, osteoarthritis, usage of blood thinner and epilepsy, although those skilled in the art will be aware of many other medical conditions that the invention can be used to monitor. The symptoms being treated or suppressed and the medication to be taken depends on the person's medical condition.

In the case of Parkinson's disease, the medication can be levodopa or a dopamine agonist, and the symptoms can be any of motor symptoms including tremors of the hands, arms, legs, jaw, face, bradykinesia (slowness of movement), rigidity, stiffness, posture instability, backward swings, freezing, micrographia (miniature handwriting), mask-like expressions, unwanted accelerations, and non-motor symptoms including the loss of the sense of smell, constipation, sleep disorders, mood disorders, depression, fatigue, perspiration (both excessive sweating (hyperhidrosis) and under secretion (hypohidrosis) and low blood pressure when standing. In addition, Parkinson's disease can lead to speech and voice disorders, such as reduced loudness, monotonous pitch, hoarseness, a 'breathy' voice and/or imprecise articulation.

In the case of depression, the effectiveness of the medication may change depending on stress, sleep, or without any apparent reason. In addition, medication for depression may take some time to start working (as in it may take several doses of the medication before symptoms start to reduce). Moreover, there may be temporary side effects (e.g. dizziness, drowsiness, nausea) with the first use or first few uses. These side effects may pass, or they may not. Changes in the person's mood as a result to medication not working can be monitored using speech and/or video analysis, as well questionnaires. It is known in the case of depression medication that for some people a particular antidepressant may simply stop working over time. The causes of this so-called "poop-out" effect or antidepressant tolerance - known as tachyphylaxis - or why it occurs in some people and not in others, is not fully understood, but the monitoring technique described herein can help to detect this.

The medication schedule for the person can be retrieved from the memory unit 6, input to the apparatus 2 by a user, e.g. the person or a care provider, or received from another device, such as a pill or medication dispenser used by the person. In particular, the medication for the person may be dispensed automatically by a pill dispenser, in which case the pill dispenser is programmed with the medication schedule for the person.

Next, based on the received medication schedule, a first monitoring interval for a first monitoring event for the person is set (step 103). A monitoring event is any event in which the condition of the person (and specifically the symptoms of the person's disease) can be monitored. A monitoring event can therefore be a telephone call between the person and another party, a video call between the person and another party, an interaction by the person with an electronic device, and/or the monitoring of the person using one or more electronic devices. In the case of a telephone call and a video call, the other party may be a person, such as a care provider (e.g. doctor), or it may be an automated service implemented by a server or computer that interacts with the person. An interaction with an electronic device can include the person operating or using a specific application on a computer, smart phone or tablet, or using a dedicated test device. For example the application can request the person complete certain activities, such as reading out phrases or sentences, performing a mental exercise, performing a physical exercise (e.g. walk using a walking stick or writing with an electronic pen), etc. In the case of the monitoring event comprising the monitoring of the person using one or more electronic devices, the electronic devices can be sensors and/or other devices, such as a camera or cameras located in the environment around the person or worn or carried by the person, that can be activated during the monitoring event in order to monitor the person.

The first monitoring interval is a time period before a first medication dose is scheduled to be taken in which a first monitoring event can be initiated. Thus the first monitoring interval defines a time window in which a first monitoring event is to take place. The first monitoring interval has a start time, an end time and a duration. The start time and end time may be defined as absolute times (e.g. 8:45am), or they may be defined relative to the time at which a medication dose is to be taken (e.g. the start time may be 20 minutes before the scheduled time for a dose of medication, and the end time may be 5 minutes before the scheduled time for a dose of medication). Generally, the duration of the monitoring interval is set based on an estimate of when symptoms of the medical condition may reoccur.

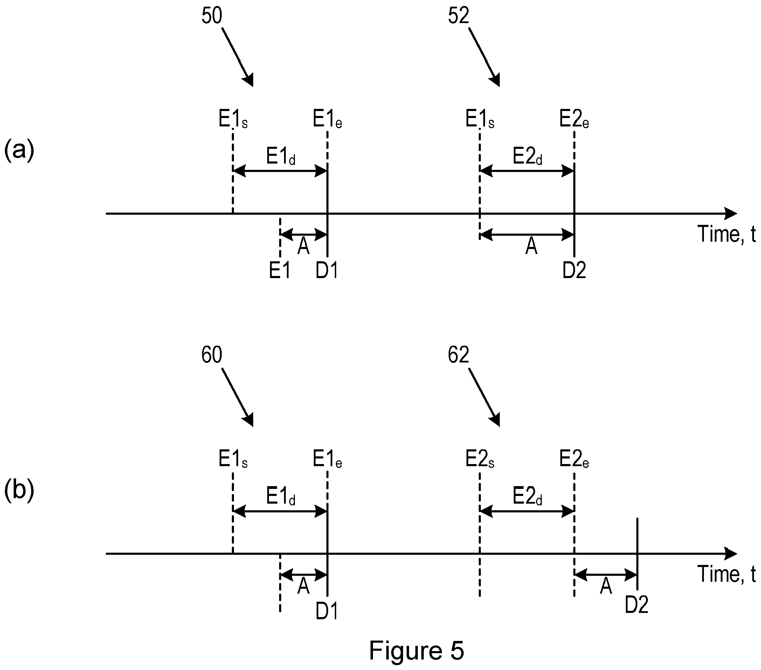

An exemplary first monitoring interval is shown in Figures 5(a) and 5(b). In both Figures, for a first medication dose, denoted D1, a first monitoring interval (labelled 50 in Figure 5(a) and labelled 60 in Figure 5(b)) is defined for a first monitoring event E1. The first monitoring interval has an end time E1ₑ set at the time at which the first medication dose D1 is to be taken, a duration E1_{d} and thus a start time E1ₛ (which is E1_{d} earlier than the end time E1ₑ). The duration E1_{d} can be any suitable length or period of time, for example 15 minutes, one hour, etc., and may be determined or set based on a number of different factors, for example the time between medication doses, (e.g. smaller durations may be used when medication doses are taken frequently, e.g. every few hours, whereas relatively longer durations may be used when medication doses are taken less frequently, e.g. once a day) and/or a time at which symptoms of the person's disease might be expected to return or become more significant.

In step 105, a first monitoring event for the person is initiated in the first monitoring interval 50/60. That is, at some time during the first monitoring interval 50/60, a first monitoring event is initiated (e.g. a call or video call is placed to the person, or the person is notified to use the relevant application on their computer, tablet or smart phone). In this step, the monitoring event can be initiated by a monitoring event device 8 being activated, initiated or otherwise used to perform a first monitoring event on the person. The exact time at which the monitoring event is started during the first monitoring interval 50/60 can be arbitrary, and may depend on the availability of the other party to make the call or video call (for example the other party may be on a call with another person at the start of the first monitoring interval 50/60, and the call to the person of interest can be initiated once the other party has completed that other call).

During the monitoring event the person is monitored for one or more characteristics associated with the one or more symptoms of the medical condition. The type of monitoring initiated in this step depends on the symptoms and the characteristics of those symptoms that can be monitored during the monitoring event. The monitoring can comprise processing an audio stream for a voice call or a video stream for a video call to identify audible and/or visual characteristics of the symptoms associated with the medical condition. For example, speech analysis can be used to identify whether the person has a speech or voice disorder such as reduced loudness, monotonous pitch, hoarseness, a 'breathy' voice and/or imprecise articulation, and in some cases speech analysis can be used to analyse the content of the person's speech, for example to identify a person's response to questions about the symptoms they may be experiencing, and/or to determine the person's mood (e.g. is the person talking negatively). As another example, a video stream can be analysed to identify whether the person has tremors, and if so the extent/magnitude of those tremors, and/or to identify other characteristics of the person's movements (e.g. which can indicate bradykinesia, rigidity, stiffness, posture, etc.). Where one or more sensors are used to monitor the person during the monitoring event, the monitoring can comprise processing a sensor measurement signal from those sensors to identify the one or more characteristics. In some embodiments, the monitoring can also or alternatively include the other party (e.g. care provider) observing the person for characteristics of the symptoms and inputting these observations into the apparatus 2. These inputs can relate to the presence, absence or severity of particular symptoms associated with the medical condition (or of the presence, absence or severity of particular characteristics associated with the symptoms).

It will be appreciated that the monitoring of the person to determine the presence or absence of the characteristics/symptoms can make use of any suitable algorithm or technique for analysing video, audio, and/or sensor measurements of the person. For example, machine learning techniques can be used to extract values for the characteristics/symptoms from the signals or measurements.

Although the event E1 is shown as a single time point in Figure 5, the event E1 typically takes some time to complete, and so the event E1 has a respective start time that is within the first monitoring interval, and a respective end time. It will be appreciated that depending on the duration of the event E1 and the time at which the event E1 is started, the event E1 may end before the time D1 that the first medication dose is scheduled to be taken, or the event E1 may end after the time D1. In the latter case, the person may therefore take the dose of medication during the monitoring event, and the effectiveness of the medication dose in starting to suppress symptoms can be directly monitored.

After the first monitoring event E1, a monitoring interval for a second monitoring event (denoted E2) is set (step 107). This monitoring interval is referred to as the second monitoring interval. As with the first monitoring interval, the second monitoring interval starts before a second medication dose is scheduled to be taken by the person and is a time period in which a second monitoring event can be initiated, and the second monitoring interval has a respective start time, end time and duration. The second monitoring event E2 will typically be of the same type as the first monitoring event E1, but in some cases the types of monitoring event can be different. For example where multiple monitoring events may be performed per day, one of the monitoring events can be a call or video call with a care provider, and another one of the monitoring events can be initiated automatically without requiring the person to perform any specific interaction (e.g. the monitoring event could involve the activation of a camera in the home of the person to record the activities of the person).

According to the invention, a start time, end time and/or duration of the second monitoring interval is determined based on the monitored one or more characteristics and the medication schedule. Thus, the timing of the second monitoring event is based on whether the person was experiencing symptoms of the medical condition during the first monitoring event.

In general, one of the aims of the invention is to identify whether, and if so, when, a person starts to experience symptoms of their medical condition (or the symptoms worsen) before the next medication dose is taken. Thus, if the first monitoring event indicates that the person is not experiencing symptoms of the medical condition, or otherwise the previous dose of medication has not yet worn off, then a subsequent monitoring event can be scheduled or set to occur the same or closer to the time at which the next medication dose is to be taken, in order to determine if the medication wears off closer to the time at which that next medication dose is taken. On the other hand, if the first monitoring event indicates that the person is experiencing symptoms of the medical condition, or in other words that the previous dose of medication is starting to wear off or has worn off, then a subsequent monitoring event can be scheduled or set to occur the same or earlier with respect to the time at which the next medication dose is to be taken than the first monitoring event, in order to determine how early the medication is starting to wear off or is wearing off.

Thus, if the monitored characteristic(s) indicate that the person did not have the one or more symptoms of the medical condition during the first monitoring event, then the start time of the second monitoring interval can be determined to be the same or closer to the time at which the second medication dose is scheduled to be taken than the start time of the first monitoring interval was to the time at which the first medication dose was scheduled to be taken. In some embodiments, the start time of the second monitoring interval can be set to a time before the second medication dose is scheduled to be taken that is equal to or less than the time before the first medication dose was scheduled to be taken that the first monitoring event ended, provided that the time that the first monitoring event ended was before the time that the first medication dose was scheduled to be taken. This is illustrated in Figure 5(a), where E1 represents the end time of the first monitoring event. If the time that the first monitoring event ended was after the time that the first medication dose was scheduled to be taken, then the start time of the second monitoring interval can be set to a time before the second medication dose is scheduled to be taken that is equal to or less than the time before the first medication dose was scheduled to be taken that the first monitoring event started. This is illustrated in Figure 5(a) if the time E1 is taken to represent the start time of the first monitoring event.

Thus, consider that the first monitoring event E1 started during the first monitoring interval 50 and ended a time amount A before the scheduled first medication dose D1. During this monitoring event E1 it was determined that the person did not have the symptoms of the medical condition or did not have the characteristics of those symptoms, and so the monitoring interval 52 for the second monitoring event E2 is set such that the second monitoring event E2 occurs no earlier than the time amount A before the time at which the second medication dose D2 is scheduled. Thus, in Figure 5(a), the start time E2ₛ of the second monitoring interval 52 is set to be the time amount A before the time at which the second medication dose D2 is scheduled to be taken. In this way, the second monitoring event E2 will occur closer to the second medication dose D2 than the end of the first monitoring event E1 was to the first medication dose D1. In this example, the end time E2ₑ of the second monitoring interval 52 is the same as the end time E1ₑ of the first monitoring interval 50 (i.e. the end time E2ₑ is the time at which the second medication dose D2 is to be taken), although in other embodiments the end time can be different to the time at which the second medication dose D2 is to be taken. However, the end time of the second monitoring interval 52 should not be later than the time at which the second medication dose D2 is to be taken.

If the monitored characteristic(s) indicate that the person did have the one or more symptoms of the medical condition during the first monitoring event, then the end time of the second monitoring interval can be determined to be the same or further from the time at which the second medication dose is scheduled to be taken than the end time of the first monitoring interval was to the time at which the first medication dose was scheduled to be taken. In some embodiments, the end time of the second monitoring interval can be set to a time before the second medication dose is scheduled to be taken that is equal to or more than the time before the first medication dose was scheduled to be taken that the first monitoring event was started. This is illustrated in Figure 5(b).

Thus, as in Figure 5(a), the first monitoring event E1 started a time amount A before the scheduled first medication dose D1. During this monitoring event E1 it was determined that the person did have the symptoms of the medical condition or did have the characteristics of those symptoms, and so the monitoring interval 62 for the second monitoring event E2 is set such that the second monitoring event E2 occurs earlier than the time amount A before the time at which the second medication dose D2 is scheduled. Thus, in Figure 5(b), the end time E2ₑ of the second monitoring interval 62 is set to be the time amount A before the time at which the second medication dose D2 is scheduled to be taken. In this way, the second monitoring event E2 will occur earlier with respect to the second medication dose D2 than the start of the first monitoring event E1 was to the first medication dose D1. In this example, the duration E2_{d} of the second monitoring interval 62 is the same as the duration E1_{d} of the first monitoring interval 60, although this does not have to be the case.

Following the setting of the second monitoring interval 52/62, the second monitoring event E2 can be initiated at some time in the second monitoring interval and the person monitored for the one or more characteristics, in a similar way to the first monitoring event E1 in step 103. That is, the monitoring event device 8 can be activated, initiated or otherwise used in this step to perform a second monitoring event on the person. Step 107 can then be repeated for a third monitoring event E3, and so on.

It will be appreciated that the first medication dose and the second medication dose do not have to be consecutive doses of medication taken by the person, although in some embodiments they are consecutive doses. For example, it may be inconvenient for a person having to take medication quite frequently (e.g. every few hours) to perform or be engaged in a monitoring event each time a dose of medication is taken. In that case, the second medication dose could be a medication dose that is on a different day to the first medication dose, for example.

In some embodiments, steps 105 and 107 can be repeated for a number of monitoring events and medication doses. The information obtained from the monitoring events (e.g. information indicating whether the person had the characteristics/symptoms of the medical condition during each monitoring event, along with information indicating the timing of the monitoring event relative to the scheduled time for taking the medication) can be used to determine the effectiveness of the medication doses in treating or suppressing the one or more symptoms of the medical condition. Those skilled in the art will be aware of suitable data processing and/or data mining techniques that can be used to determine an effectiveness of a medication dose based on the information obtained during these monitoring events.

In some embodiments, step 105 can further comprise determining a severity of the one or more symptoms based on the monitored one or more characteristics. For example, some symptoms may be present or absent, whereas other symptoms can have varying levels of severity (e.g. low, moderate and severe). In this case, when the second monitoring interval is being set in step 107, the duration of the second monitoring interval can be set based on the determined severity. In particular, if the symptoms in the first monitoring event are quite severe, then this is an indication that the next monitoring event needs to occur much earlier to identify when the medication starts to wear off, and so the duration should be increased, and for example set to a relatively large value. Thus, the duration of the second monitoring interval can be set longer than the duration of the first monitoring interval if the severity of the one or more symptoms is above a threshold. On the other hand, the duration of the second monitoring interval can be set the same as a duration of the first monitoring interval if the severity of the one or more symptoms is below the threshold. Those skilled in the art will be aware of suitable data processing techniques that can be used determine the severity of a symptom (e.g. a magnitude of tremor movements, etc.), and thus further details are not provided herein.

In some embodiments, the actual time at which the person took a dose of medication can be used in step 107 to set the second monitoring interval. Thus, for example, if the person took the dose earlier than scheduled, then the medication may wear off earlier than anticipated just from observing the medication schedule, and likewise if the person took the dose later than scheduled, then the medication may wear off later than anticipated just from observing the medication schedule. Therefore the apparatus 2 can receive information indicating a time at which the person took the first medication dose from the person themselves (e.g. as a user input into the apparatus 2 or monitoring event device 6), or from a pill or medication dispenser used by the person. In this case, the start time, end time and/or duration of the second monitoring interval can be determined based on the monitored one or more characteristics, the medication schedule and the time at which the person took the first medication dose. Alternatively or in addition, the information on the time at which the person took the first dose of medication can be used to interpret the characteristics/symptoms observed during the second monitoring event. For example, if the characteristics/symptoms are present during a monitoring event (or are severe) and the person took the last dose of medication early, then this might (at least in part) explain the presence of the symptoms, and so the next monitoring event may not necessarily need to be moved earlier relative to the next dose of medication. On the other hand, if the characteristics/symptoms are not present during a monitoring event when they would otherwise be expected to be present, and the person took the last dose of medication late, then this might (at least in part) explain the absence of the symptoms, and so the next monitoring event may not necessarily need to be moved later relative to the next dose of medication.

In some embodiments, the information collected during a monitoring event (and preferably during multiple monitoring events) can be used to determine whether to adjust the medication schedule (e.g. the timing and/or dosage of medication doses). Preferably the information indicating when the person actually took the doses of medication is also used as part of this determination. For example, if during monitoring events a person always or often has symptoms of the medical condition, then it may be appropriate for the medication schedule to be adjusted to increase the medication dosage. In this case, the apparatus 2 could provide an output to a care provider indicating the medication schedule might or should be adjusted, and the care provider can take the appropriate action. Alternatively the apparatus 2 could determine the adjustment to the medication schedule automatically (for example within certain rules and/or guidelines established for the particular medication, person and/or medical condition) and output this to the care provider for approval. As another alternative, the apparatus 2 could determine the adjustment to the medication schedule automatically and provide the adjusted medication schedule to a pill or medication dispenser to automatically effect the adjustment.

If the medication schedule is adjusted (whether manually by a care provider or automatically by the apparatus 2), then the duration of the next monitoring interval (whether the second monitoring interval or a subsequent monitoring interval) can be increased relative to a previous monitoring interval to increase the speed (i.e. in terms of the number of monitoring events required) with which any negative effects can be detected. The duration of the next monitoring interval may also be increased relative to a previous monitoring interval if some other significant event occurs in the life of the person. Alternatively, if the medication schedule is adjusted, the start time, end time and/or duration of the next monitoring interval can be reset to default values.

The following description relates to specific embodiments of the invention that are applied to Parkinson's disease, but it will be appreciated that many of the described features can be used more generally for other medical conditions.

In these specific embodiments the monitoring event is a video call between a care provider and a person, with calls occurring regularly, for example every day for around 10 minutes each time. The person has a medication schedule for levodopa, which for example may indicate that the person is to take levodopa every four hours at 8am, 12pm, 4pm, etc.

A call scheduler engine (which is implemented by the processing unit 4 in the apparatus 2) receives the medication schedule, and can optionally receive an indication of the call frequency (i.e. how often and/or for which or how many doses of medication a call is required) and/or a desired call duration. Based on the medication schedule, the call scheduler engine outputs a schedule for call events. This schedule can be a time interval in which the next call is to be made, or it can be a schedule indicating time intervals for several medication doses indicating when calls should be made. In the latter case, after a monitoring event (call) the call scheduler engine will update the schedule for subsequent calls based on whether symptoms were present or not.

An example of the operation of the call scheduler engine (processing unit 4) according to steps 101-107 of Figure 4 is set out below.

It is assumed that calls are performed every day, and that the PD (Parkinson's disease) person takes levodopa every 4 hours, for example at 8am, 12pm, 4pm and 8pm. Candidate times for calls are 8 am, 12 pm, 4 pm and 8 pm, and the person indicates a preference for calls around 12 pm. The first call is scheduled to be in the following interval (the first monitoring interval 50/60):
interval_start = drug_intake - on_off_max,
interval_end = drug_intake
Call interval: [interval_start interval_end]
where "drug_intake" is the time that the next medication dose is scheduled and "on_off_max" is a parameter that determines the step size, that is, how fast the call times can change. The initial values of this parameter can be based on population statistics. In this example, this parameter is static (i.e. constant). However, as described before, the parameter value can be dynamically adapted according to the observed PD symptoms.

A call is then randomly placed at a time in this interval, referred to as "call_time", corresponding to the start of the monitoring event. During the call the person is observed for any visible PD symptoms. The analysis of PD symptoms can happen automatically using video and/or speech analysis, and/or manually by the care provider.

If no 'off' state PD symptoms are observed (i.e. the person is in an 'on' state), that is if the drug is working as intended, then the lower boundary of the call interval (the interval start time) is moved to be equal to the current call time. Thus,
interval_start = call_time;
which means that all subsequent calls will happen at the same or later time.

If 'off' state PD symptoms are observed, then the current time needs to be modified to be earlier because it is desired to find out what the time range is where the current drug dosage is working, so that this information can be used to update the drug regimen.

The time interval for subsequent calls is updated as follows. The upper boundary of the interval (the interval end time) is set to the current call time so that calls cannot happen later than this time. The lower boundary of the interval (the start time) is calculated from the end time using the on_off_max parameter.

Although in some embodiments on_off_max is constant, in additional embodiments on_off_max can be adapted based on PD symptom severity. If PD symptoms during the call are many then the call should have happened at a much earlier time, so the on_off_max is set to have a higher values. If the symptoms are minor, then there is no need to make subsequent calls too early. Thus,
interval_end = call_time
interval_start = interval_end - on_off_max

In some cases it might be difficult to decide if the PD person is in 'off' state. In this case, the call interval is adapted to be similar to the case above (i.e. where PD symptoms are observed), but with a smaller step size. As an example, on_off_max = 10.

Some exemplary code that can be used to implement the above operations, and certain exemplary embodiments, is set out below. In this example, the monitoring event is represented by the call time, and for the sake of simplicity it is assumed that it is of negligible duration, in other words event start time ≈ event end time.

```
 ***********************************************************************
 %example code for call scheduling
 clear all;
 drug intake = 720; %12pm in minutes, 00:00 = 0 minutes
 on_off_max = 20; %pre-set parameter: maximum call interval, can be
 based on population statistics, can also vary depending on PD severity
 on off min = 10; %pre-set parameter: intermediate call interval, for
 making smaller steps
 interval_start = drug_intake - on_off_max;
 interval_end = drug_intake;
 pd_symptoms = [0, 0, 0, 0, 2, 2, 1, 1, 0, 0, 0, 1, 0, 0, 0, 2, 1, 1, 0, 0, 0]; %0=none,
 1=observed, 2=no sure (or maybe)
 output = [];
 for i=1:length (pd_symptoms)
     pd_symp = pd_symptoms(i);
     %pick a time between interval start and interval end, randomly
 call time = randi([interval_start, interval_end],100,1);
     call time = call_time(50);
      
     %record the call times, and observed symptom
     output = cat(1,output,[call_time,
 interval_start,interval_end,pd_symp]);
     %set the new interval
     if pd symp == 0 %no PD symptom observed --> move the call time
 closer to drug intake time
          interval_start = max(call _time, interval_start);
     elseif pd_symp == 1 %PD symptom observed --> move the call time
 earlier than current call time, and make sure that subsequent calls
 will be no later than current call time
          interval_end = min(call_time,interval_end);
          interval_start = interval_end-on_off_max;
     else %not sure, or maybe --> similar to pd_symp = 1, but update
     with a smaller step size
          interval_end = min(call_time,interval_end);
          interval_start = interval_end-on_off_min;
     end
     %additional embodiment
     %if the observed PD symptom severity is too high, this is an
 indication
     %that the call need to happen much earlier. so in this case, the
 step
     %size defined my on off max can be increased. An example
 implementation can be as follows:
     %if pd_severity > thereshold
     %on_off_max = on_off_max + 5; %in this case we dynamically adapt
 the
     %step size based on the observed PD severity
     %end
 end
 ***********************************************************************
```

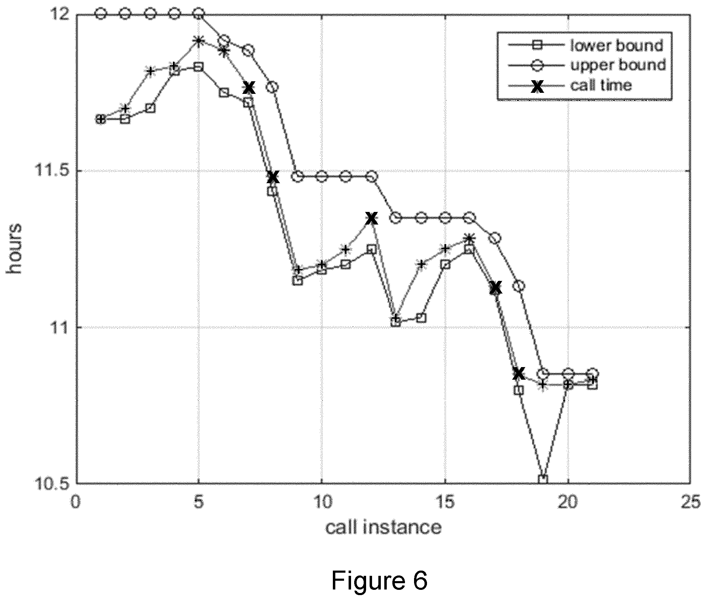

The graph in Figure 6 illustrates the operation of the call scheduler described above. The graph shows how the monitoring interval changes over time as monitoring events (video calls) are performed. In this example, a scheduled medication dose is at 12pm each day, and 21 monitoring events are performed. The graph shows the upper bound (the end time of the interval), the lower bound (the start time of the interval) and when a call is placed. For each call the symbol indicates whether no symptoms were observed (marked with a '+'), whether symptoms were observed (marked with a 'X') and whether symptoms might have been observed (marked with a '*'). It can be seen that if no symptoms are observed the call time moves closer to the dose time, and if symptoms are observed then the calls start to occur earlier.

As noted above, during each call the video and audio data from the person is analysed to identify characteristics or symptoms of PD. In addition to processing the video and audio data, measurements from other sensors can also be used if available. For example measurements of heart rate (HR), heart rate variability (HRV), respiration and person movement by sensors can be also analysed to extract PD symptoms. However in other embodiments features such as HR, HRV and respiration can be extracted from video signals using techniques known in the art.

Analysis of video and speech is a well-established area, and it is relatively straightforward to extract features that are relevant for the PD symptoms analysis. For example it is possible to detect faces and extract face markers, and extract how these markers change over time. Similarly, using speech analysis it is possible to detect difficulty with speaking, articulation, and comprehension.

The analysis of the video and audio can be performed in real time during the call, in which case the care provider can be provided with an indication of the characteristics or symptoms of the person. This may allow the care provider to query the person on these symptoms or to ask the person to perform tests to ascertain the extent of these symptoms. Alternatively, the analysis can be performed once the call is completed.

As noted above, the care provider may provide their input on the characteristics and/or symptoms of the person, and this input may be obtained by the apparatus 2 requesting the user to complete a questionnaire that is completed during or following the call.

Once several calls have been performed, a decision can be made as to whether to change the drug regimen, and if so, how to change it. Typically this will be done by a care provider such as a doctor. Therefore, some or all of the information gathered during the calls will be made available to the doctor.

In some embodiments, the characteristics relating to the PD symptoms are visualised (i.e. plotted as graphs and figures) and presented to the doctor. For example, the characteristics can include the amount of tremor, speech tempo and pitch (related to intelligibility). The apparatus 2 may evaluate many additional characteristics, and the care provider can select the ones to view and evaluate. The care provider may also be able to review video and/or audio recordings of the person (e.g. as obtained during the call).

Likewise, the characteristics relating to the PD symptoms can be visualised (i.e. plotted as graphs and figures) and presented to the person to show the person the progression if their condition. The information presented to the person can also include advice relating to the medical condition, such as perform specific types of physical exercise (e.g. take a walk or use a bike), or speech exercises (e.g. singing) which are known to have benefits for tremors and speech related improvements, respectively.

In addition to tremors and speech related characteristics/symptoms, the processing unit 4 can analyse the video stream to determine the level of sweating of the person. Excessive or increased sweating is one of the important PD symptoms, and can be identified automatically from a video stream using techniques known in the art.

As noted above, the monitoring event can typically be a video call or voice call. However, in other embodiments, a camera installed at the person's home can be automatically activated at the times determined by the call scheduler/processing unit 4. The data collected by the camera can be analysed as discussed above to determine the presence of characteristics/symptoms of the medical condition. Although the data collected in this way may lack the quality of the data collected during interactions with a care provider, it can still provide useful information on the effectiveness of the medication. In some cases, the camera can be part of the pill/medication dispenser.

In some embodiments, where the person takes the medication several times per day, the processing unit 4 can schedule monitoring events for different ones of those doses of medication each day to identify whether there might be interactions between the medication and food or other activities. For example, the call scheduler may alternate every day between two drug intake times (e.g. 12 pm and 4 pm) to get interaction effects due to food and other user activities.

As noted above, it is not necessary to perform a monitoring event with the person for each dose of medication that they are to take. An optimal event frequency (i.e. an event frequency that minimises the number of interactions required with the person but that enables the effectiveness of the medication to be evaluated over short and longer-term timescales) can be determined based on historical data for the person, and also data from other people. In some embodiments, people can be profiled or categorised) according to their symptoms, and this information is used to determine the call frequency. One particular implementation is described below.

Firstly, a person specific feature set is calculated that consists of person data (e.g. age, sex, height, weight) and characteristics obtained from physiological measurements, such as characteristics from accelerometer signals, characteristics for speech, characteristics from skin (e.g. temperature, sweat).

The calculated features are then used to cluster the people, for example by employing machine learning techniques either in a supervised or unsupervised manner. Clustering of the data is preferred as it is more accurate. In the supervised method, some of the data points (i.e. people) are labelled and it is known how they should be treated and/or communicated with, for example with what frequency events should occur.

A different event frequency can then be assigned for different clusters, where the frequency correlates with symptom severity. In case of the supervised learning, the person information for that cluster can be used to determine the frequency.

It will be appreciated that although the method and apparatus are described above as being for monitoring a particular medical condition, if a person has multiple medical conditions being treated by the same or a different medication, the method and apparatus can be used to monitor the effectiveness of the treatment of that or those other conditions during the monitoring events by extracting characteristics relating to the symptoms of that/those other conditions.

There is therefore provided a method and apparatus that provides improvements in the scheduling of monitoring events in which the person is monitored for symptoms of a disease and that enables better decisions to be taken on a medication regimen for the person, and also (or alternatively) enables information to be collected on the effectiveness of a medication regimen.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method of monitoring a person, the method comprising:
(i) receiving medication schedule information regarding the person, the medication schedule information indicating the timing of one or more medication doses for treating or suppressing one or more symptoms of a medical condition;
(ii) setting a first monitoring interval for a first monitoring event for the person based on the received medication schedule, wherein the first monitoring interval starts before a first medication dose is scheduled to be taken in which a first monitoring event can be initiated;
(iii) initiating a first monitoring event for the person in the first monitoring interval and monitoring the person for one or more characteristics associated with the one or more symptoms of the medical condition during the first monitoring event;
(iv) setting a second monitoring interval for a second monitoring event for the person, wherein the second monitoring interval starts before a second medication dose is scheduled to be taken by the person in which a second monitoring event can be initiated, and wherein a start time, end time and/or duration of the second monitoring interval is determined based on the monitored one or more characteristics and the medication schedule.

2. A computer-implemented method as claimed in claim 1, wherein if the monitored one or more characteristics indicate that the person did not have the one or more symptoms of the medical condition during the first monitoring event, the start time of the second monitoring interval is determined to be (i) the same amount of time before the time at which the second medication dose is scheduled to be taken than the start time of the first monitoring interval was to the time at which the first medication dose was scheduled to be taken, or (ii) closer to the time at which the second medication dose is scheduled to be taken than the start time of the first monitoring interval was to the time at which the first medication dose was scheduled to be taken.

3. A computer-implemented method as claimed in claim 1 or 2, wherein if the monitored one or more characteristics indicate that the person did have the one or more symptoms of the medical condition during the first monitoring event, the end time of the second monitoring interval is determined to be (i) the same amount of time before the time at which the second medication dose is scheduled to be taken than the end time of the first monitoring interval was to the time at which the first medication dose was scheduled to be taken, or (ii) further from the time at which the second medication dose is scheduled to be taken than the end time of the first monitoring interval was to the time at which the first medication dose was scheduled to be taken.

4. A computer-implemented method as claimed in any of claims 1-3, wherein the method further comprises the step of:
determining a severity of the one or more symptoms based on the monitored one or more characteristics; and
wherein the duration of the second monitoring interval is determined based on the determined severity of the one or more symptoms.

5. A computer-implemented method as claimed in claim 4, wherein the duration of the second monitoring interval is set longer than a duration of the first monitoring interval if the severity of the one or more symptoms is above a first threshold.

6. A computer-implemented method as claimed in claim 4 or 5, wherein the duration of the second monitoring interval is set the same as a duration of the first monitoring interval if the severity of the one or more symptoms is below a second threshold.

7. A computer-implemented method as claimed in any of claims 1-6, wherein the step of monitoring the person for one or more characteristics comprises processing an audio and/or video signal, and/or a sensor measurement signal obtained during the first monitoring event to determine the one or more characteristics.

8. A computer-implemented method as claimed in any of claims 1-7, wherein the step of monitoring the person for one or more characteristics associated with the one or more symptoms of the medical condition comprises receiving an input from a party involved in the monitoring event indicating the presence, absence and/or severity of the one or more symptoms during the first monitoring event.

9. A computer-implemented method as claimed in any of claims 1-8, wherein the method further comprises the step of:
receiving information indicating a time at which the person took the first medication dose.

10. A computer-implemented method as claimed in any of claims 1-8, wherein the method further comprises the step of:
estimating a time at which the person took the first medication dose from measurements of the one or more characteristics associated with the one or more symptoms of the medical condition.

11. A computer-implemented method as claimed in claim 9 or 10, wherein the start time of the second monitoring interval, and/or the end time of the second monitoring interval and/or duration of the second monitoring interval is determined based on (i) the monitored one or more characteristics, (ii) the medication schedule and (iii) the time at which the person took the first medication dose.

12. A computer-implemented method as claimed in any of claims 1-11, wherein the duration of the first monitoring interval is set based on an estimate of when the one or more symptoms may occur following an earlier medication dose.

13. A computer-implemented method as claimed in any of claims 1-12, wherein the monitoring event is a telephone call, a video call, an interaction by the person with an electronic device, or the monitoring of the person using one or more electronic devices.

14. A computer program product comprising a computer readable medium having computer readable code embodied therein, the computer readable code being configured such that, on execution by a suitable computer or processor, the computer or processor is caused to perform the method of any of claims 1-13.

15. An apparatus for monitoring a person, the apparatus comprising:
a processing unit configured to:
receive medication schedule information regarding the person, the medication schedule information indicating the timing of one or more medication doses for treating or suppressing one or more symptoms of a medical condition;
set a first monitoring interval for a first monitoring event for the person based on the received medication schedule, wherein the first monitoring interval starts a time period before a first medication dose is scheduled to be taken in which a first monitoring event can be initiated;
initiate a first monitoring event for the person in the first monitoring interval and monitoring the person for one or more characteristics associated with the one or more symptoms of the medical condition during the first monitoring event; and
set a second monitoring interval for a second monitoring event for the person, wherein the second monitoring interval starts a time period before a second medication dose is scheduled to be taken by the person in which a second monitoring event can be initiated, and wherein a start time, end time and/or duration of the second monitoring interval is determined based on the monitored one or more characteristics and the medication schedule.
